(19) Europäisches Patentamt
European Patent Office
Office européen des brevets

(11) EP 0 807 099 B1

(12) **EUROPÄISCHE PATENTSCHRIFT**

(45) Veröffentlichungstag und Bekanntmachung des Hinweises auf die Patenterteilung:
**15.07.1998 Patentblatt 1998/29**

(21) Anmeldenummer: **96901747.4**

(22) Anmeldetag: **22.01.1996**

(51) Int Cl.[6]: **C07C 51/58**, C07C 63/70

(86) Internationale Anmeldenummer:
**PCT/EP96/00237**

(87) Internationale Veröffentlichungsnummer:
**WO 96/23758 (08.08.1996 Gazette 1996/36)**

(54) **VERFAHREN ZUR HERSTELLUNG VON KERNHALOGENIERTEN BENZOYLCHLORIDEN**

PROCESS FOR PREPARING NUCLEUS-HALOGENATED BENZOYL CHLORIDES

PROCEDE DE PREPARATION DE CHLORURES DE BENZOYLE HALOGENES AU NOYAU

(84) Benannte Vertragsstaaten:
**BE CH DE FR GB IE IT LI LU NL**

(30) Priorität: **03.02.1995 DE 19503471**

(43) Veröffentlichungstag der Anmeldung:
**19.11.1997 Patentblatt 1997/47**

(73) Patentinhaber: **BAYER AG**
**51368 Leverkusen (DE)**

(72) Erfinder:
- **GEHRING, Reinhold**
  **D-42327 Wuppertal (DE)**
- **MÜLLER, Herbert**
  **D-52372 Kreuzau (DE)**
- **HARDENBICKER, Georg**
  **D-51688 Wipperfürth (DE)**
- **BUSSMANN, Werner**
  **D-51373 Leverkusen (DE)**

(56) Entgegenhaltungen:
**EP-A- 0 166 939** **DE-A- 4 301 247**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft ein besonderes vorteilhaftes Verfahren zur Herstellung von kernhalogenierten Benzoylchloriden aus den entsprechenden Benzotrichloriden.

Aus der DE-A 4 301 247 ist bekannt, daß man bestimmte kernhalogenierte Benzoylchloride herstellen kann, indem man die entsprechenden kernhalogenierten Benzotrichloride mit Wasser in Gegenwart von Eisen(III)-chlorid partiell verseift. Nachteilig bei diesem Verfahren ist, daß korrosive Reaktionsgemische gehandhabt werden müssen (z.B. Wasser, Salzsäure, Eisensalze und gegebenenfalls Fluoridionen enthaltende Flüssigkeiten). Deshalb müßten bei einer technischen Anwendung dieses Verfahrens häufig Reaktionsgefäße aus Sonderwerkstoffen eingesetzt werden. Außerdem treten häufig durch Autokatalyse hervorgerufene starke Exothermien auf, sodaß besondere Maßnahmen zu deren Beherrschung notwendig sind.

Es wurde nun ein Verfahren zur Herstellung von kernhalogenierten Benzoylchloriden gefunden, das dadurch gekennzeichnet ist, daß man kernhalogenierte Benzotrichloride mit Ameisensäure in Gegenwart von Eisensalzen umsetzt.

In das erfindungsgemäße Verfahren kann man beispielsweise fluorierte und/oder chlorierte Benzotrichloride einsetzen. Vorzugsweise setzt man Benzotrichloride der Formel (I) ein

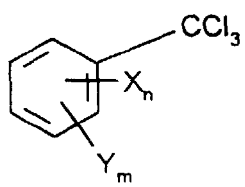

(I),

in der

X für Fluor und Y für Wasserstoff oder Chlor stehen und

n eine ganze Zahl von 1 bis 5 und m = 5-n bedeuten

Besonders bevorzugte Benzotrichloride sind 2,4-Dichlor-5-fluor-, 2,3,5,6-Tetrafluor-, 2,3,4-Trifluor-5-chlor- und 2,3,4-Trifluor-benzotrichlorid, insbesondere bevorzugt ist 2,3,4,5-Tetrafluorbenzotrichlorid. Einsetzbare kernhalogenierte Benzotrichloride sind bekannte Verbindungen oder analog den bekannten Verbindungen herstellbar.

Ameisensäure kann man in das erfindungsgemäße Verfahren z.B. in Mengen von 0,8 bis 1,1 Mol pro Mol kernhalogeniertes Benzotrichlorid einsetzen. Vorzugsweise beträgt diese Menge 0,95 bis 1,05 Mol, besonders bevorzugt 1 Mol.

Als Eisensalz kommt insbesondere Eisen(III)-chlorid in Frage. Eisensalze kann man z.B. in Mengen von 0,05 bis 3 Gew.-%, bezogen auf kernhalogeniertes Benzotrichlorid einsetzen. Vorzugsweise beträgt diese Menge 1 bis 2 Gew.-%.

Das erfindungsgemäße Verfahren kann man z.B. bei Temperaturen im Bereich 20 bis 100°C durchführen. Bevorzugt sind Temperaturen im Bereich 40 bis 70°C.

Der Druck, bei dem das erfindungsgemäße Verfahren durchgeführt wird, ist nicht kritisch. Man kann bei Unterdruck, Normaldruck oder Überdruck arbeiten. Vorzugsweise arbeitet man bei Normaldruck.

Geeignete Werkstoffe für Apparate zur Durchführung des erfindungsgemäßen Verfahrens sind z.B. Edelstähle.

Während der erfindungsgemäßen Umsetzung entweichen aus dem Reaktionsgemisch pro Mol umgesetzter Ameisensäure 1 Mol Kohlenmonoxid und 2 Mol Chlorwasserstoff. Den Chlorwasserstoff kann man in Wasser absorbieren und die dabei erhältliche Salzsäure für die verschiedensten Zwecke weiterverwenden. Das Kohlenmonoxid kann man einer Verbrennung zuführen.

Die erfindungsgemäße Umsetzung läuft schnell ab. Das bedeutet, man kann Ameisensäure so zudosieren, wie sie abreagiert. Den Verbrauch an Ameisensäure kann man durch Kontrolle des Kohlenmonoxid-Gehaltes im Abgas verfolgen.

Nach Durchführung des erfindungsgemäßen Verfahrens liegt im allgemeinen ein Reaktionsgemisch vor, daß im wesentlichen das hergestellte kernhalogenierte Benzoylchlorid und eine Eisensalze enthaltende Masse enthält. Aus dem Reaktionsgemisch kann man das hergestellte kernhalogenierte Benzoylchlorid z.B. durch Destillation abtrennen. Die dann hinterbleibende Eisensalze enthaltende Masse kann anstelle von frischen Eisensalzen bei einem weiteren Ansatz wieder verwendet werden. Bei mehrmaliger Rückführung der Eisensalze enthaltenden Masse ist es vorteilhaft, von Zeit zu Zeit eine kleine Menge frischer Eisensalze, insbesondere Eisen(III)-chlorid hinzuzufügen, z.B. nach jeder zweiten bis fünften Rückführung 10 bis 50 Gew.-% der ursprünglich eingesetzten Menge Eisensalze.

Kernhalogenierte Benzoylchloride, wie sie auf erfindungsgemäße Art besonders gut zugänglich sind, sind wichtige Zwischenprodukte zur Herstellung von antiinfektiv wirkenden Arzneimitteln (siehe z.B. DE-A 3 420 770 und EP-A 417 669).

Das erfindungsgemäße Verfahren hat eine Reihe von Vorteilen. Es kann in Apparaten aus üblichen Werkstoffen durchgeführt werden, da bei ihm weniger korrosive Reaktionsgemische anfallen als bei bekannten Verfahren. Weiterhin kann man mit Hilfe des erfindungsgemäßen Verfahrens kernhalogenierte Benzoylchloride in Ausbeuten von meist über 90 % herstellen, und man kann es dosierkontrolliert ablaufen lassen.

**Beispiele**

Prozentangaben sind Gewichtsprozente, soweit nichts anderes vermerkt ist.

**Beispiel 1**

2,3,5,6-Tetrafluorbenzoylchlorid

268 g 2,3,5,6-Tetrafluorbenzotrichlorid wurden in einem 0,5 1 Vierhalskolben vorgelegt. Nach Zugabe von 5,4 g Eisen(III)-chlorid wurde unter Rühren auf 60°C erhitzt. Bei 60 - 65°C wurden in 6 Stunden 46 g Ameisensäure zudosiert. Es setzte sofort eine starke Gasentwicklung (Kohlenmonoxid, Chlorwasserstoff) ein. Der Gasstrom wurde über einen Kühler einem Absorptionsturm zugeführt. Nach vollständiger Dosierung wurde noch zwei Stunden bei 60°C nachgerührt. Anschließend wurde das Produkt im Vakuum destilliert.

Es wurden 12,9 g Vorlauf (GC-.-Analyse: 99,1 %) und 163,5 g Hauptlauf (Kp: 54 - 56°C/20 mbar; GC-Analyse: 99,2 %) erhalten. Die Gesamtausbeute betrug 83 % d. Th.

**Beispiel 2**

4-Fluorbenzoylchlorid

In einer Apparatur bestehend aus einem 1 l-Vierhalskolben mit Rührer, Thermometer, Rückflußkühler und Tropftrichter wurden 300 g 4-Fluorbenzotrichlorid und 1,5 g Eisenchlorid vorgelegt. Die Mischung wurde auf 80°C aufgeheizt.

Dann wurden insgesamt 50 ml Ameisensäure innerhalb von 5 Stunden so zudosiert, daß die Temperatur gut gehalten werden konnte. Danach lagen 220 g eines Gemisches mit folgender Zusammensetzung vor:

| | |
|---|---|
| 89,8 % | 4-Fluorbenzoylchlorid |
| 1,5 % | 4-Fluorbenzotrichlorid |
| 2,1 % | 4-Fluorbenzoesäureanhydrid |
| 6,6 % | Unbekannte |

Hieraus wurden durch fraktionierte Destillation 185 g 4-Fluorbenzoylchlorid mit einem Gehalt von 99,1 % (83 % d. Th.) erhalten. Der Destillationsrückstand enthielt überwiegend 4-Fluorbenzotrichlorid und kann in den nächsten Ansatz zurückgeführt werden.

**Beispiel 3**

3-Fluorbenzoylchlorid

In einer Apparatur bestehend aus einem 1 1-Vierhalskolben mit Rührer, Thermometer, Rückflußkühler und Tropftrichter wurden 300 g 3-Fluorbenzotrichlorid und 1,5 g Eisenchlorid vorgelegt. Die Mischung wurde auf 80°C aufgeheizt. Dann wurden insgesamt 53 ml Ameisensäure innerhalb von 5 Stunden so zudosiert, daß die Temperatur gut gehalten werden konnte.

Danach lagen 217 g eines Gemisches mit folgender Zusammensetzung vor:

| | |
|---|---|
| 92,4 % | 3-Fluorbenzoylchlorid |
| 2,3 % | 3-Fluorbenzotrichlorid |
| 1,7 % | 3-Fluorbenzoesäureanhydrid |
| 3,6 % | Unbekannte |

Hieraus wurden nach fraktionierter Destillation 190 g 3-Fluorbenzoylchlorid mit einem Gehalt von 99,3 % (85 % d. Th.) erhalten. Der Destillationsrückstand enthielt überwiegend 3-Fluorbenzotrichlorid und kann in den nächsten Ansatz zurückgeführt werden.

**Beispiel 4**

2,3,4,5-Tetrafluorbenzoylchlorid

a) In einer Rührapparatur wurden 268 g 2,3,4,5-Tetrafluorbenzotrichlorid vorgelegt und 5,3 g Eisen (III)-chlorid zugegeben. Bei 60°C wurden in 8 Stunden 45 g Ameisensäure (98 - 100 %) eingepumpt. Es setzte sofort eine starke Gasentwicklung (Kohlenmonoxid, Chlorwasserstoff) ein. Der Gasstrom wurde über einen Kühler einem Absorptionsturm zugeführt. Nach vollständiger Dosierung wurde bis zum Ende der Gasentwicklung gerührt. Anschließend wurde das Produkt destilliert. Es wurden 195 g 2,3,4,5-Tetrafluorbenzoylchlorid erhalten (91,5 % d. Th.); Kp: 70 - 72°C/20 mbar, $n_D^{25}$: 1,4773.

b) Zum Destillationsrückstand (ca. 20 g) wurden 268 g 2,3,4,5-Tetrafluorbenzotrichlorid gegeben und 2,7 g Eisen(III)-chlorid zugesetzt. Analog a) wurde die Mischung mit Ameisensäure umgesetzt. Es wurden 198 g (93 % d. Th.) 2,3,4,5-Tetrafluorbenzoylchorid erhalten.

**Patentansprüche**

1. Verfahren zur Herstellung von kernhalogenierten Benzoylchloriden, dadurch gekennzeichnet, daß man kernhalogenierte Benzotrichloride mit Ameisensäure in Gegenwart von Eisensalzen umsetzt.

2. Verfahren nach Anspruch 1, dadurch gekennzeichnet, daß man als kernhalogenierte Benzotrichloride solche der Formel (I) einsetzt,

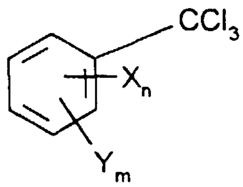

(I),

in der

X für Fluor und Y für Wasserstoff oder Chlor stehen und

n eine ganze Zahl von 1 bis 5 und m = 5-n bedeuten.

3. Verfahren nach Ansprüchen 1 und 2, dadurch gekennzeichnet, daß man 0,8 bis 1,1 Mol Ameisensäure pro Mol kernhalogeniertes Benzotrichlorid einsetzt.

4. Verfahren nach Ansprüchen 1 bis 3, dadurch gekennzeichnet, daß man als Eisensalz Eisen(III)-chlorid einsetzt.

5. Verfahren nach Ansprüchen 1 bis 4, dadurch gekennzeichnet, daß man Eisensalze in einer Menge von 0,05 bis 3 Gew.-%, bezogen auf kernhalogeniertes Benzotrichlorid, einsetzt.

6. Verfahren nach Ansprüchen 1 bis 5, dadurch gekennzeichnet, daß man es bei Temperaturen im Bereich 20 bis 100°C durchführt.

7. Verfahren nach Ansprüchen 1 bis 6, dadurch gekennzeichnet, daß man den während der Umsetzung entweichenden Chlorwasserstoff in Wasser absorbiert und das während der Umsetzung entweichende Kohlenmonoxid einer Verbrennung zuführt.

8. Verfahren nach Ansprüchen 1 bis 7, dadurch gekennzeichnet, daß man aus dem nach der Umsetzung vorliegenden Reaktionsgemisch das hergestellte kernhalogenierte Benzoylchlorid durch Destillation abtrennt.

9. Verfahren nach Ansprüchen 1 bis 8, dadurch gekennzeichnet, daß man aus dem nach der Umsetzung vorliegenden Reaktionsgemisch das hergestellte kernhalogenierte Benzoylchlorid durch Destillation abtrennt und die hinterbleibende Eisensalze enthaltende Masse anstelle von frischen Eisensalzen bei einem weiteren Ansatz wieder verwendet.

**Claims**

1. Process for the preparation of ring-halogenated benzoyl chlorides, characterized in that ring-halogenated benzotrichlorides are reacted with formic acid in the presence of iron salts.

2. Process according to Claim 1, characterized in that the ring-halogenated benzotrichlorides used are those of the formula (I)

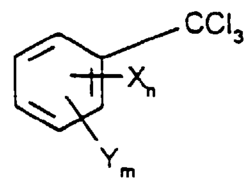

(I),

in which

X represents fluorine and Y represents hydrogen or chlorine and

n denotes an integer from 1 to 5 and m = 5-n.

3. Process according to Claims 1 and 2, characterized in that 0.8 to 1.1 mol of formic acid is used per mole of ring-halogenated benzotrichloride.

4. Process according to Claims 1 to 3, characterized in that the iron salt used is iron(III) chloride.

5. Process according to Claims 1 to 4, characterized in that iron salts are used in an amount of 0.05 to 3% by weight, based on ring-halogenated benzotrichloride.

6. Process according to Claims 1 to 5, characterized in that it is carried out at temperatures in the range 20 to 100°C.

7. Process according to Claims 1 to 6, characterized in that the hydrogen chloride escaping during the reaction is absorbed in water and the carbon monoxide escaping during the reaction is burnt.

8. Process according to Claims 1 to 7, characterized in that the ring-halogenated benzoyl chloride prepared is separated off by distillation from the reaction mixture present after the reaction.

9. Process according to Claims 1 to 8, characterized in that the ring-halogenated benzoyl chloride prepared is separated off by distillation from the reaction mixture present after the reaction and the mixture containing iron salts remaining is reused in a further batch instead of fresh iron salts.

## Revendications

1. Procédé pour la préparation de chlorures de benzoyle halogénés au noyau, caractérisé en ce qu'on fait réagir des benzotrichlorures halogénés au noyau avec de l'acide formique en présence de sels de fer.

2. Procédé selon la revendication 1, caractérisé en ce qu'on met en oeuvre, à titre de benzotrichlorures halogénés au noyau, ceux répondant à la formule (I)

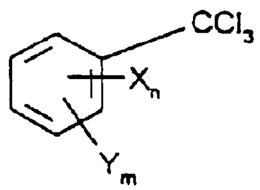

(I),

dans laquelle

X représente un atome de fluor et Y représente un atome d'hydrogène ou un atome de chlore, et

n représente un nombre entier de 1 à 5 et m = 5-n.

3. Procédé selon les revendications 1 et 2, caractérisé en ce qu'on met en oeuvre de 0,8 à 1,1 mole d'acide formique par mole de benzotrichlorure halogéné au noyau.

4. Procédé selon les revendications 1 à 3, caractérisé en ce qu'on met en oeuvre, à titre de sel de fer, du chlorure de fer(III).

5. Procédé selon les revendications 1 à 4, caractérisé en ce qu'on met en oeuvre des sels de fer en une quantité de 0,05 à 3% en poids rapportés au benzotrichlorure halogéné au noyau.

6. Procédé selon les revendications 1 à 5, caractérisé en ce qu'on l'effectue à des températures dans le domaine de 20 à 100°C.

7. Procédé selon les revendications 1 à 6, caractérisé en ce qu'on absorbe dans de l'eau le gaz chlorhydrique qui s'échappe au cours de la mise en réaction et on achemine à une combustion le monoxyde de carbone qui s'échappe au cours de la mise en réaction.

8. Procédé selon les revendications 1 à 7, caractérisé en ce qu'on sépare par distillation, hors du mélange réactionnel présent après la mise en réaction, le chlorure de benzoyle obtenu halogéné au noyau.

9. Procédé selon les revendications 1 à 8, caractérisé en ce qu'on sépare par distillation, hors du mélange réactionnel présent après la mise en réaction, le chlorure de benzoyle obtenu halogéné au noyau, et on réutilise dans une charge ultérieure la masse contenant les sels de fer qui subsistent au lieu de sels de fer frais.